# EUROPEAN PATENT APPLICATION

(11) **EP 4 086 358 A1**
(43) Date of publication of application: **09.11.2022**
(21) Application number: 21382416.2
(22) Date of filing: 07.05.2021
(51) Int. Cl.: C12Q 1/6883, G01N 33/50

(54) **METHOD FOR THE PROGNOSIS OF PATIENTS SUFFERING FROM ATRIAL FIBRILLATION**

(71) Applicant: Fundación Instituto de Investigación Sanitaria De Santiago de Compostela (FIDIS), Santiago de Compostela (ES); Servizo Galego de Saúde (SERGAS), 15703 Santiago de Compostela (ES)
(72) Inventor: EIRAS PENAS, Sonia, 15706 A Coruña (ES); RODRÍGUEZ-MAÑERO, Moisés, 15706 A Coruña (ES)
(74) Representative: Hoffmann Eitle

(57) **Abstract**

The present invention refers to a method for predicting the response of patients suffering from atrial fibrillation (AF) to catheter ablation treatment, or for the prognosis of patients suffering from AF.

## Description

### FIELD OF THE INVENTION

The present invention pertains to the medical field. Particularly, the present invention refers to a method for predicting the response of patients suffering from atrial fibrillation (AF) to catheter ablation treatment, or for the prognosis of patients suffering from AF.

### STATE OF THE ART

Diverse pathological processes affecting the atria contribute to the genesis and perpetuation of AF. The term 'atrial cardiomyopathy' has been introduced to describe changes affecting the atria with potential clinical implications and a histological/pathophysiological classification scheme has been proposed to outline the dominant underlying pathology. However, nowadays, little is known regarding the best way to characterize atrial cardiomyopathy. Tissue fibrosis can be detected and quantified as low voltage area (LVA) below specific selected thresholds at the time of the electroanatomic mapping (EAM). Although the measured voltage is influenced by several factors, atrial cardiomyopathy assessment can be inferred at the time of AF ablation. This underlying LVA is relevant first in terms of a modulator of the strategy to be implemented and second as a marker of success after AF catheter ablation procedures. It has been previously proposed that pulmonary vein isolation (PVI) only is enough if the identified LVAs does not exceed 10% of the left atrial (LA) surface area. A major limitation is that this information is not available until the procedure and other non-invasive methods for determining the extent of atrial fibrosis are limited.

Although some strategies have been previously suggested to reflect the level of atrial fibrosis, conflicting evidence exists regarding the utility of biomarkers in predicting the presence of atrial cardiomyopathy, at least determined by EAM, and their relevance in terms of recurrence after AF ablation.

So, there is an unmet medical need of finding reliable strategies focused on predicting the response of patients suffering from AF to catheter ablation treatment, or on prognosing patients suffering from AF, as an indication of the possible presence of underlying fibrotic scar and arrhythmia recurrence after catheter ablation treatment.

The present invention aims at solving this problem and a method for predicting the response of patients suffering from AF to catheter ablation treatment, or for the prognosis of AF, is herein provided.

### DESCRIPTION OF THE INVENTION

### Brief description of the invention

As explained above, the present invention refers to a method for predicting the response of patients suffering from AF to catheter ablation treatment, or for the prognosis of patients suffering from AF.

In general terms, the inventors of the present invention have tested the ability of circulating biomarkers such as Galectin-3 (Gal-3) and Fatty acid binding protein 4 (FABP4) to identify the presence of LVAs in the EAM with the intention of assessing their role in the prediction of AF recurrence after catheter ablation. Gal-3 is a protein that in humans is encoded by the LGALS3 gene. It is a member of the lectin family, of which 14 mammalian galectins have been identified. FABP4 is a master regulator of the transcription factor peroxisome proliferator-activated receptor gamma (PPARG) involved on adipocyte differentiation and, in consequence, a marker of adipose tissue inflammatory activity.

Specifically, blood samples were obtained from the left atrium and peripheral vein of consecutive patients referred for radiofrequency ablation. Bipolar voltage map was created. Areas of bipolar voltage < 0.5 mV were considered as a LVA. Patients were classified into two groups LVA <10% or ≥10%. Logistic regression analysis was performed to study independent predictors for LVA. A score was achieved considering AF type (1,2,3 for paroxysmal, persistent and long-standing persistent AF), peripheral FABP4 levels (>20 ng/mL) and peripheral Gal-3 (>10 ng/mL). The Kaplan-Meier was used to describe freedom from recurrent AF for the categorical scores. Comparison of survival curves among categorical scores was performed using a log-rank test. Cox proportional hazard model was used to evaluate the effect of these categorical scores on freedom from AF recurrent.

Three hundred sixteen patients, 100 (32%) women, mean age 58±10 were included. AF was paroxysmal in 103 (33%), persistent in 131 (41%) and long-standing in 82 (27%). 73 presented LVA ≥10%. Multivariate analysis revealed gender (female), atrial or peripheral Gal-3 and FABP4 levels, LAA and AF type (long-standing persistent pattern) independent predictors for LVA ≥10%. During a mean follow-up of 655±494 days freedom of recurrence was 60%. The model including AF type+FABP4+Gal-3 stratified patients regarding freedom from recurrence, ranging from 27% (1 point) to 88% (5 points). Cox analysis determined that AF type+FABP4+Gal-3 were able to discriminate the population according the highest risk for AF recurrence after ablation (OR 5.82; CI95% 1.95-17.39; p<0.001).

So, the present invention has a clear clinical relevance since the results herein provided demonstrate that peripheral FABP4 and Gal-3 levels along with AF type were able to reclassify patients according to atrial myopathy/presence of LA fibrosis along with low or high risk of AF recurrent. As a matter of fact, we have identified a subgroup of patients by combining these markers (FAB4, Gal-3, presence of LVA ≥ 10% and AF pattern) in whom the success rate was probably unreasonable (below 25%). Under our point of view these findings might be of interest not only for a better selection of patients referred to AF ablation but also for identifications of patients that might benefit from closer follow-up.

Consequently, it can be concluded that markers of adiposity (FABP4) and fibrosis (Gal-3) can be of particular interest in patients with persistent AF. A mechanistic *"in vitro"* assay showed the effect of high FABP4 levels on human atrial fibroblasts proliferation. These findings are of interest not only for a better selection of patients but also for a better understanding of the mechanism leading to AF, key to find alternative therapies.

So, the first embodiment of the present invention refers to an *in vitro* method for predicting the response of patients suffering from AF to catheter ablation treatment which comprises: a) Measuring the concentration level of at least [Gal-3 and FABP4] in a biological sample obtained from the patient, and b) wherein if the concentration level of at least [Gal-3 and FABP4] is statistically higher as compared with a pre-established threshold concentration level, it is an indication that the patient will not respond to catheter ablation treatment.

The second embodiment of the present invention refers to an *in vitro* method for the prognosis of patients suffering from AF which comprises: a) Measuring the concentration level of at least [Gal-3 and FABP4] in a biological sample obtained from the patient, and b) wherein if the concentration level of [Gal-3 and FABP4] is statistically higher as compared with a pre-established threshold concentration level, it is an indication that the patient has a bad prognosis, wherein bad prognosis indicates the presence of underlying fibrotic scar and arrhythmia recurrence after catheter ablation treatment.

The third embodiment of the present invention refers to the *in vitro* use of at least [Gal-3 and FABP4] for predicting the response of patients suffering from AF to catheter ablation treatment, or for the prognosis of patients suffering from AF.

In a preferred embodiment, the present invention is carried out in persistent AF patients in which a catheter ablation is indicated for treatment.

The fourth embodiment refers to a kit comprising: a) Reagents or tools for obtaining a minimally-invasive sample from the patients (preferably plasma, blood or serum), and b) Reagents or tools for measuring the concentration level of at least [Gal-3 and FABP4].

The fifth embodiment refers to the use of the kit for predicting the response of patients suffering from AF to catheter ablation treatment, or for the prognosis of patients suffering from AF.

In a preferred embodiment, the present invention comprises: a) Measuring the concentration level of at least [Gal-3 and FABP4] in a biological sample obtained from the subject, b) processing the concentration level values in order to obtain a risk score and c) wherein if the concentration level is statistically higher as compared with a pre-established threshold concentration level, it is an indication that the patient will not respond to catheter ablation treatment or has a bad prognosis.

In a preferred embodiment, the present invention is carried out in patient suffering from AF.

In a preferred embodiment, the present invention is carried out in minimally-invasive biological samples.

In a preferred embodiment, the present invention is carried out in plasma, blood or serum.

In a preferred embodiment, the present invention refers to a method for treating AF patients by carrying out catheter ablation, which comprises a previous step of predicting the response of patients suffering from AF to catheter ablation treatment, by following the method of the invention explained above which comprises a) Measuring the concentration level of at least [Gal-3 and FABP4] in a biological sample obtained from the patient, and b) wherein if the concentration level of at least [Gal-3 and FABP4] is statistically higher as compared with a pre-established threshold concentration level, it is an indication that the patient will not respond to catheter ablation treatment, or c) wherein if the concentration level of at least [Gal-3 and FABP4] is statistically lower as compared with a pre-established threshold concentration level, it is an indication that the patient will respond to catheter ablation treatment.

In a preferred embodiment, the present invention is a computer-implemented invention, wherein a processing unit (hardware) and a software are configured to:
- Receive the concentration level values of the above cited biomarkers or signatures,
- Process the concentration level values received for finding substantial variations or deviations, and
- Provide an output through a terminal display of the variation or deviation of the concentration level, wherein the variation or deviation of the concentration level indicates that the patients suffering from AF may respond or not to catheter ablation treatment.

In a preferred embodiment, the present invention refers to a method for detecting the concentration level of [Gal-3 and FABP4] in a test sample from a patient suffering from AF in order to determine whether the patient may respond or not to catheter ablation treatment, which comprises performing an immunoassay in a biological sample obtained from the patient.

For the purpose of the present invention the following terms are defined:
- The expression "minimally-invasive biological sample" refers to any sample which is taken from the body of the patient without the need of using harmful instruments, other than fine needles used for taking the blood from the patient, and consequently without being harmfully for the patient. Specifically, minimally-invasive biological sample refers in the present invention to: blood, serum, or plasma samples.
- The expression "pre-established threshold concentration level", refer to a "reference value" of the concentration level of the proteins based on mean values of the analysed population.
- The expression "risk score" refers to a risk value obtained after processing one or more concentration values into a single value (or risk value). This risk value will be compared with a reference value to evaluate if the AF patient might respond to catheter ablation treatment.
- By "comprising" it is meant including, but not limited to, whatever follows the word "comprising". Thus, use of the term "comprising" indicates that the listed elements are required or mandatory, but that other elements are optional and may or may not be present.
- By "consisting of' it is meant "including, and limited to", whatever follows the phrase "consisting of'. Thus, the phrase "consisting of' indicates that the listed elements are required or mandatory, and that no other elements may be present.

### Description of the figures

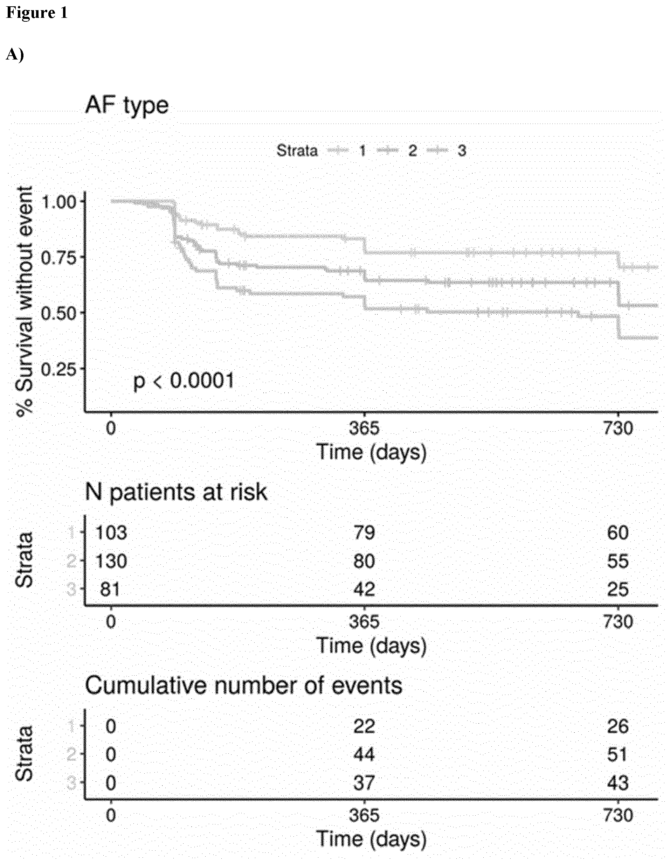

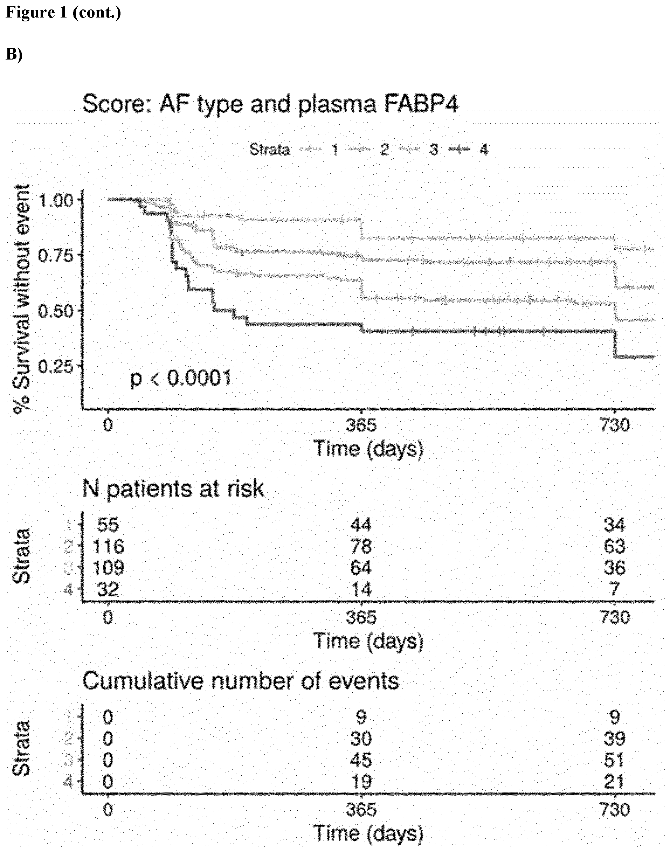

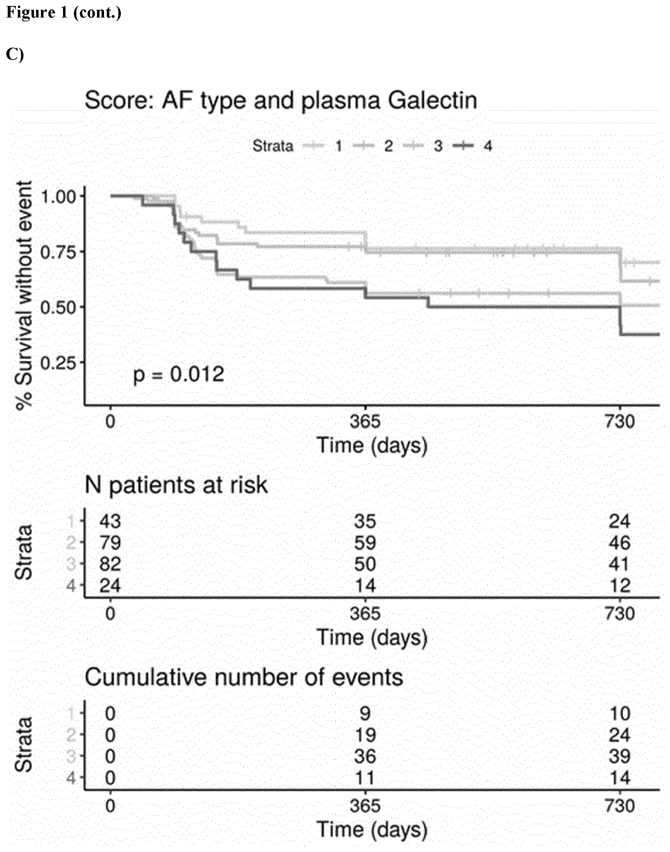

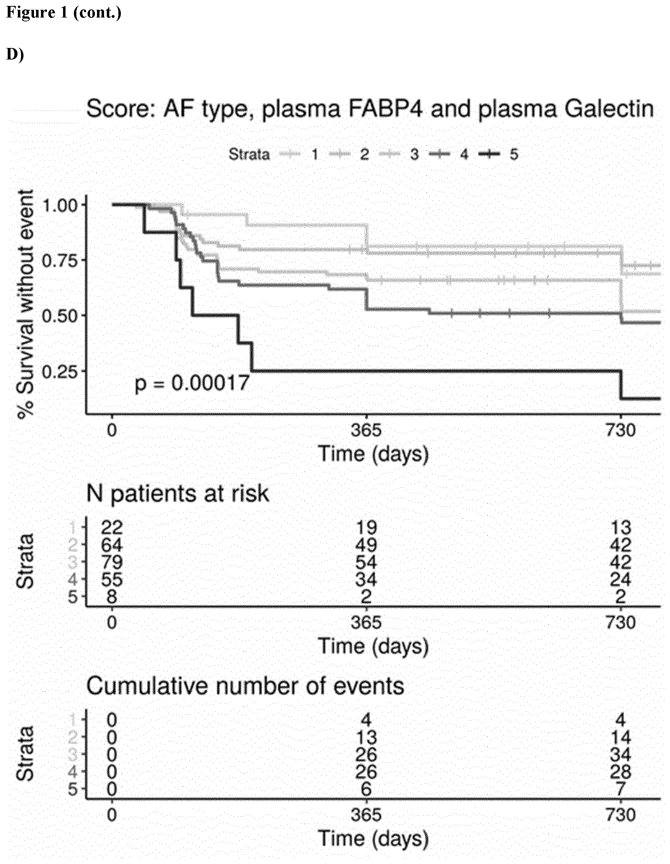

**Figure 1****. Kaplan-Meier curves with scores estimating freedom from AF after pulmonary vein isolation: a)** Score considering AF type, p<0,001 **b)** Score considering the combined AF type + peripheral FABP4 levels p<0,001 **c)** Score considering combined AF type + peripheral Gal-3 levels, p<0,001 **d)** Score considering combined AF type+ peripheral FABP4 + peripheral Gal-3 levels, p<0,001.

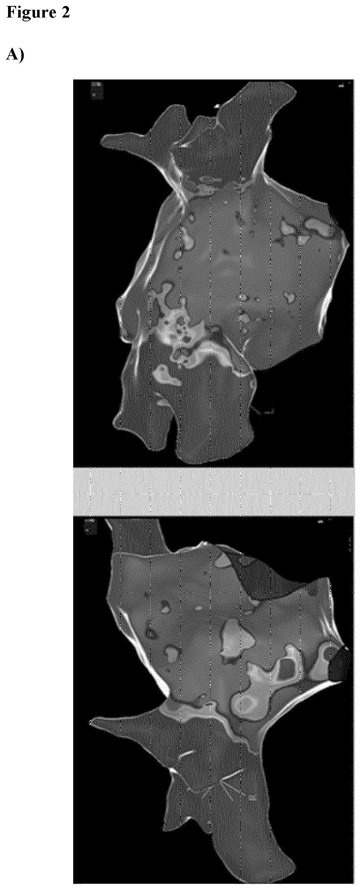

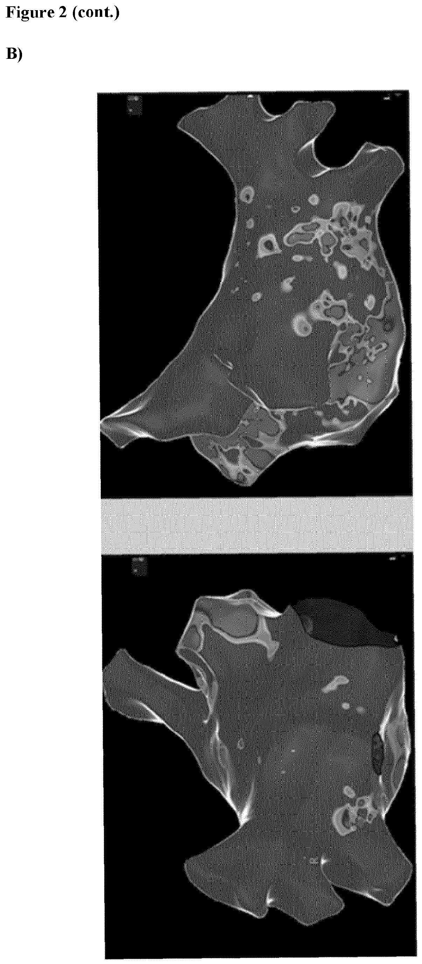

**Figure 2****. Electroanatomial map of the left atrial from two female patients with differential peripheral FABP4 and Galectin 3 levels. A) Patient 1:** Woman, 68 years old. Obese (BMI 32). HBP. Persistent AF (3 years). LA area 23 cm². eGFR = 86 mL/min/1.73m2. FABP=19,6 ng/mL. Galectin 3 = 3,35 ng/mL. **B) Patient 2:** Woman, 70 years old. Obese (BMI 31). HBP, DM-2 (HbA1c 7,3%). Persistent AF (9 months). LA area 24 cm². eGFR = 56 mL/min/1.73m2. FABP= 55,7 ng/mL. Galectin 3 = 21,16 ng/mL.

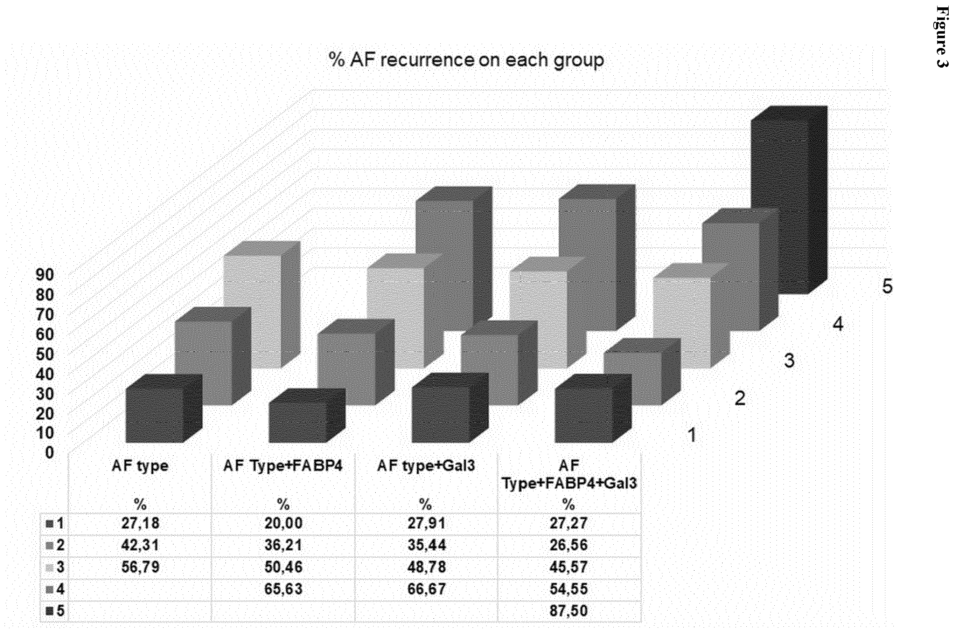

**Figure 3****. Percentage of AF recurrence on each group according to the combine score of AF pattern (+1,2,3 points) plus peripheral FABP4 (+1) and/or peripheral Galectin 3 levels >10 ng/mL (+1).** Score 1 is referred to paroxysmal AF, score 2 is referred to persistent AF or paroxysmal AF with FABP4 levels > 20 ng/mL or with Gal-3 levels > 10 ng/mL, score 3 is referred to long-standing persistent AF or persistent AF with FABP4 levels > 20 ng/mL or with Gal-3 levels > 10 ng/mL, score 4 is referred to persistent AF with FABP4 levels > 20 ng/mL and Gal-3 levels > 10 ng/mL or long-standing persistent AF with FABP4 levels > 20 ng/mL or with Gal-3 levels > 10 ng/mL and score 5 is refereed to long-standing persistent AF with FABP4 levels > 20 ng/mL and Gal-3 levels > 10 ng/mL.

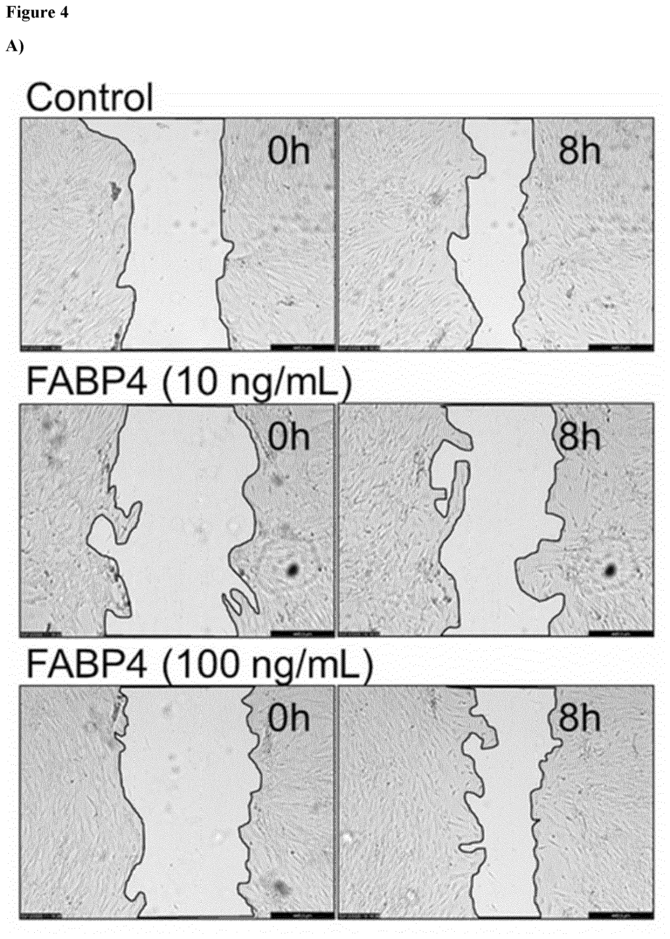

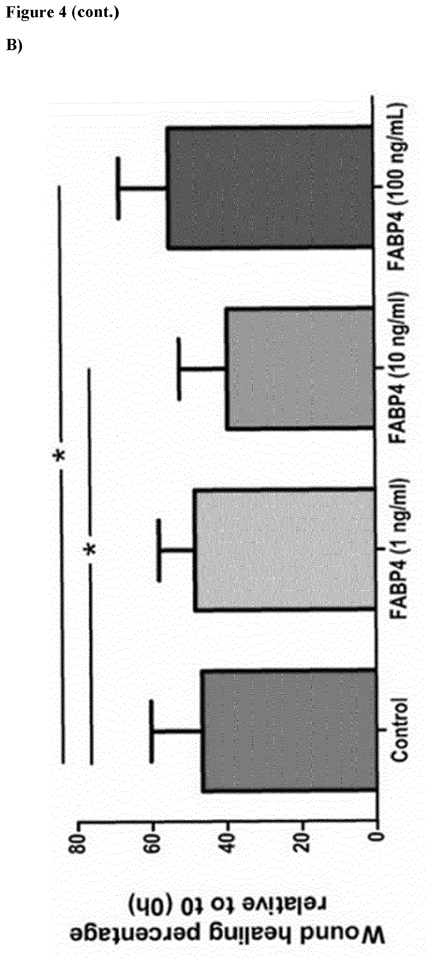

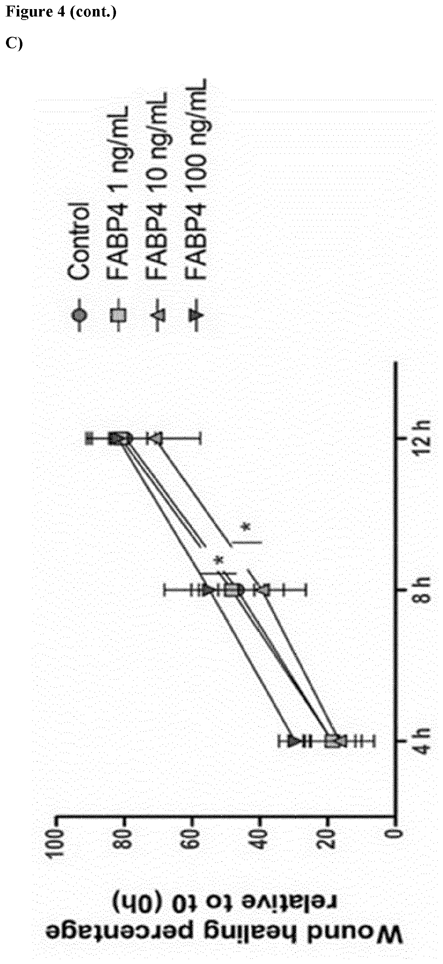

**Figure 4****. FABP4 effect on wound healing of human atrial fibroblasts with foetal bovine serum (FBS) determines proliferation assay. A)** Optical microscopy of wound healing on human atrial fibroblasts. **B)** Graph bars represents mean±SD of proliferation area, in arbitrary units, with FABP4 ng/mL (1, 10 and 100 ng/mL). **C)** Time course of proliferation assay for 4-, 8- or 12-hours on human atrial fibroblasts.

### Detailed description of the invention

The present invention is illustrated by means of the Examples set below without the intention of limiting its scope of protection.

### Example 1. Material and methods

### Example 1.1. Subjects

From September 2016, through September 2020, a total of 316 consecutive patients with paroxysmal, persistent or long-standing persistent AF, classified according clinical practice guidelines, referred for pulmonary vein (PV) radiofrequency catheter ablation in a single center were included in the study. The exclusion criteria were age under 18 years, pregnancy and any latent infectious condition. All of the patients signed the informed consent. The study protocol follows the ethical guidelines of Declaration of Helsinki and approved by Ethical Committee of Clinical Research of our region according to Helsinki Declaration.

### Example 1.2. Blood sample collection

Before the ablation procedure (after a night of fasting), immediately after the transeptal puncture and before heparin administration, blood samples were obtained from the left atrium (LA) through the transeptal sheath. At the same time, a peripheral blood sample was obtained from an ante-cubital vein using an 18-G butterfly cannula with a two-syringe technique, discarding the first 5 mL of blood and using the second 5 mL for measures. LA and peripheral blood samples were collected in EDTA-tubes.

### Example 1.3. Plasma measurements

After centrifuging at 1800xg for 10 minutes, the atrial and peripheral plasma samples were stored at -80 ° C until used. A magnetic Luminex multiplex test kit (R&D Systems, MN, USA) was used for analyzing FABP4 and leptin levels. The manufacturer's instructions were followed when analyzing plasma levels of FABP4 and leptin. The sensitivity for FABP4 and Leptin was 95.7 and 10.2 pg/mL respectively. Galectin-3 levels were determined using a commercially available enzyme-linked immunosorbent assay (ELISA) kit (BMS279-4; eBioscience, Vienna, Austria), according to the manufacturers' protocols. Measurements were performed in duplicate, and the results were averaged. The intra-assay and inter-assay coefficients of variation were 7.5% and 5.4%, respectively.

### Example 1.4. Ablation procedure and patient follow-up

Patients were undergoing point-by-point radiofrequency catheter ablation (with contact force sensing technology, SmartTouch, Biosense Inc.). The procedural endpoint was ipsilateral PVI. Most of the patients were discharged 24-36 hours after the procedure. Oral anticoagulation (OA) was maintained for at least 3 months (until the first medical visit). Then, OA was continued lifelong in those patients with a CHA₂DS₂-VASc score of ≥ 1 in men or ≥ 2 in women. During the blanking period (3 months), it is the standard of care in our center to continue or restart previously antiarrhythmic drug therapy (ADT). If the patient is free of recurrence after these 3 months, as evidenced by clinical evaluation and 24h Holter recording, patients are encouraged to discontinue ADT and only restart them in case of relapse. In case of a second recurrence after ADT or electrical cardioversion if needed, and always outside the blanking period, patients are advised for a redo ablation procedure. Medical visits were systematically performed at 3, 6, 12 and 24 months after the index procedure. Each visit comprised detailed history, physical examination and 12-lead electrocardiogram (ECG).

### Example 1.5. Left atrial low-voltage areas

Bipolar voltage map was created simultaneously with LA surface reconstruction, guided by a three-dimensional EAM system (CARTO3, Biosense Webster) using a multipolar mapping catheter (PentaRay, Biosense Webster). Patients in AF rhythm at the start of the procedure systematically underwent electrical cardioversion in the electrophysiology laboratory after the transeptal puncture. Adequate quality of the acquired voltage points was established according to CONFIDENSE module after respiratory compensation. This is a continuous mapping software with automated data acquisition when set-criteria are met, among them: 1) tissue proximity indication (proximity-based filtering of points acquired with the PentaRay); 2) wavefront annotation (an automated annotation algorithm that incorporates both unipolar and bipolar signals); 3) map consistency (system identifies outlying points when certain criteria are met; 4) position stability filter (ensures catheter position is consistent with previous location, settled in < 5 mm); 5) cycle length stability (keeps data collected within a range of predefined cycle lengths, settled in 10% over the average). A minimum number of points were requested (>1000) and the density fill threshold remained constant at ≤5 mm.

Contiguous areas of bipolar voltage < 0.5 mV were considered as a LVAs in sinus rhythm. Total LA surface area was defined as the LA body area without the PV antrum regions, LA appendage orifice, and mitral valve. Medians of the total LA surface area and area of each predefined region were measured offline on the three-dimensional reconstructed LA model. Median values of LVAs were set in relation to the surface area of each region and the entire LA.

### Example 1.6. Outcome's definition

### Presence of low-voltage area on electroanatomic mapping

We classified patients into two groups according to the extent of the LVA <10% or ≥10% of the total LA surface.

### Recurrence after AF catheter ablation

Recurrence was defined as electrocardiographic or Holter-documented AF, atrial flutter, or atrial tachycardia > 30 seconds occurring beyond the blanking period (3 months) after the procedure.

### Example 1.7. FABP4 effects on human atrial fibroblasts (wound healing)

Atrial normal human cardiac fibroblasts (NHCF-A) (Lonza, Porriño, Spain) were seeded at approximately 1 × 10⁵ cells per well in 6-multiwell plates. Cells were starved for 8 hours. Afterwards, a would field was performed by a plastic pipette tip of 0.2 mL, with a defined gap of 1 mm in each well. Then, cells were treated with FABP4 (Cloud Clone, corp. CCC, USA) at 1, 10 or 100 ng/mL, or vehicle (3 wells per condition), with foetal bovine serum (FBS) at 10% to test migration or proliferation effects, respectively. Random fields were photographed with a microscope (Leica DMI6000B Automated/Motorized Inverted Microscope, Leica Microsystems, Wetzlar, Germany). Then area at the edge of the lesion was quantified at different times (0, 4, 8, 12, 16 and 24 h) and analysed by ImageJ software (National Institutes of Health, Bethesda, Maryland, USA, https://imagej.nih.gov/ij/, 1997-2018). The experiment was performed by triplicate in NHCF-A between 4-6 passages

### Example 1.8. Statistical analyses

Categorical variables were represented as percentage and continues variables as mean ± standard deviation (SD) or interquartile range according their normal or scatter distribution, tested by Kolmogorov-Smirnov. Population was classified according AF type and Kruskal-Wallis or chi-squared tests were used for analyzing differences among continues or categorical variables, respectively. Logistic regression analysis was performed to study independent predictors for LVA < or > than 10%. A score was achieved considering the addition of the following points: AF type (1,2,3 for paroxysmal, persistent and long-standing persistent AF, respectively), peripheral FABP4 levels >20 ng/mL (1) and peripheral Gal-3 > 10 ng/mL (1). Score 1 is referred to paroxysmal AF, score 2 is referred to persistent AF or paroxysmal AF with FABP4 levels > 20 ng/mL or with Gal-3 levels > 10 ng/mL, score 3 is referred to long-standing persistent AF or persistent AF with FABP4 levels > 20 ng/mL or with Gal-3 levels > 10 ng/mL, score 4 is referred to persistent AF with FABP4 levels > 20 ng/mL and Gal-3 levels > 10 ng/mL or long-standing persistent AF with FABP4 levels > 20 ng/mL or with Gal-3 levels > 10 ng/mL and score 5 is refereed to long-standing persistent AF with FABP4 levels > 20 ng/mL and Gal-3 levels > 10 ng/mL. Similar score was performed with LVA > 10% (1). The Kaplan-Meier was used to describe freedom from recurrent AF for the categorical scores performed with the combined of each parameter (AF type, AF type+Gal-3, AF type+FABP4, AF type+Gal-3+FABP4 with or without LVA parameter). Time-0 corresponds to the moment of PVI. Comparison of survival curves among categorical scores was performed using a log-rank test. Cox proportional hazard model was used to evaluate the effect of these categorical scores on freedom from AF recurrent. P values were penalized by Benjamini-Hochberg. All analyses were performed with Statistical Package for Social Sciences (SPSS) software (SPSS, Inc., Chicago, Ill.) and p < 0.05 was considered with statistical significance.

### Example 2. Results

### Example 2.1. Population characteristics

Three hundred sixteen patients, 100 (32%) women, with a mean age of 58±10 years were included. AF pattern was paroxysmal in 103 (33%), persistent in 131 (41%) and long-standing persistent in 82 (27%) patients. The mean body mass index (BMI) was 29.6±4.3 kg/m². The left atrial area (LAA) average was 20 ± 6 cm². The LVA presented a median of 1.08 % (0.06-9.98). The median and interquartile range of Gal-3 levels were similar between atrial 9.2 (5.8-13.8) ng/mL and peripheral 10.1 (6.3-14.9) ng/mL. The median and interquartile range of FABP4 levels were higher in peripheral 18.9 (11.6-27.9) ng/mL than in atrial samples 15.8 (9.4-24.5) ng/mL. Most of patients were taking oral anticoagulants (direct oral anticoagulants 62% vs vitamin K antagonists 36%). About 69% of patients were receiving beta-blockers at the time of the ablation. Mean follow-up was 655±494 days and 290 patients completed more than 12 months of follow-up. Main baseline characteristics are shown on **Table 1**

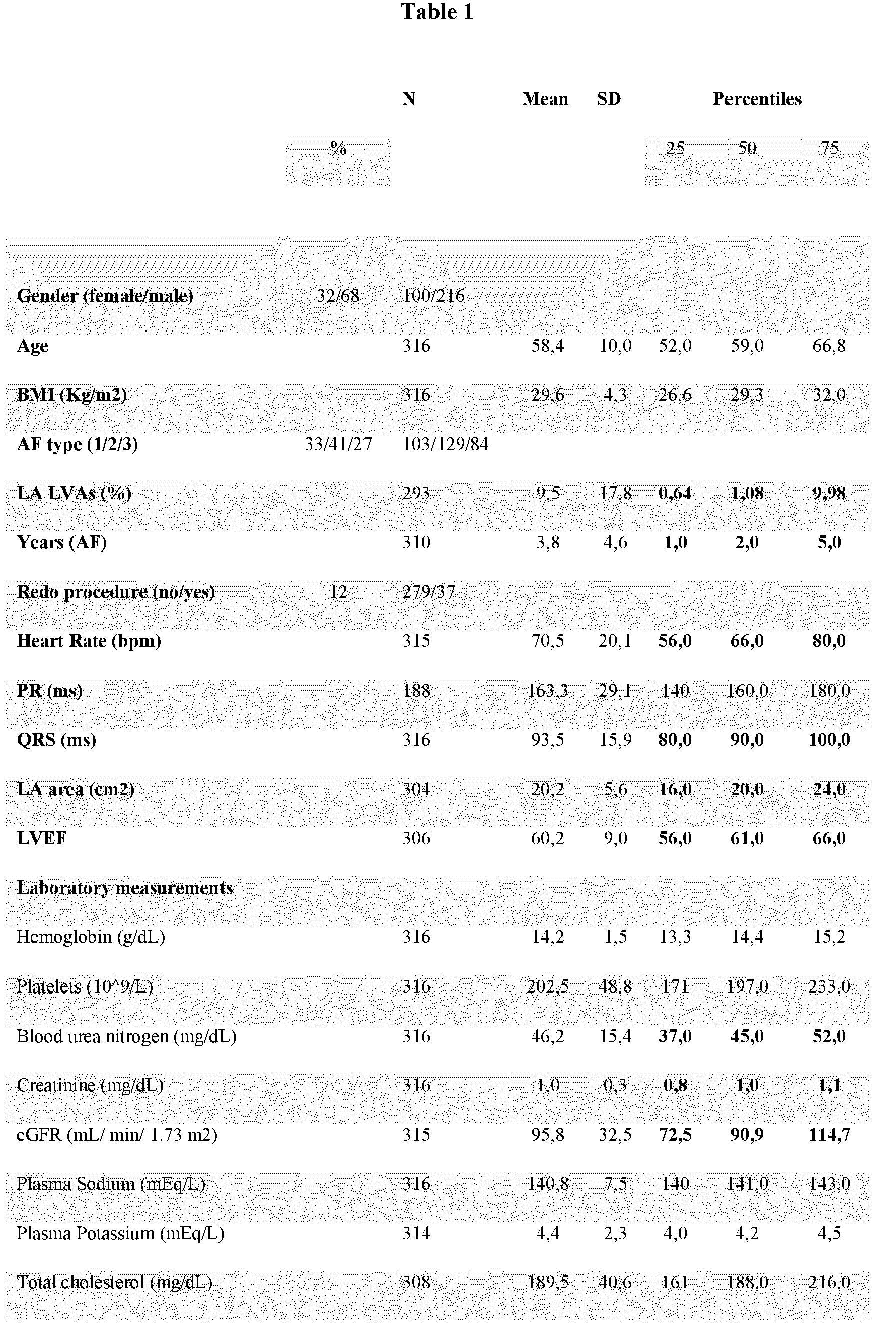

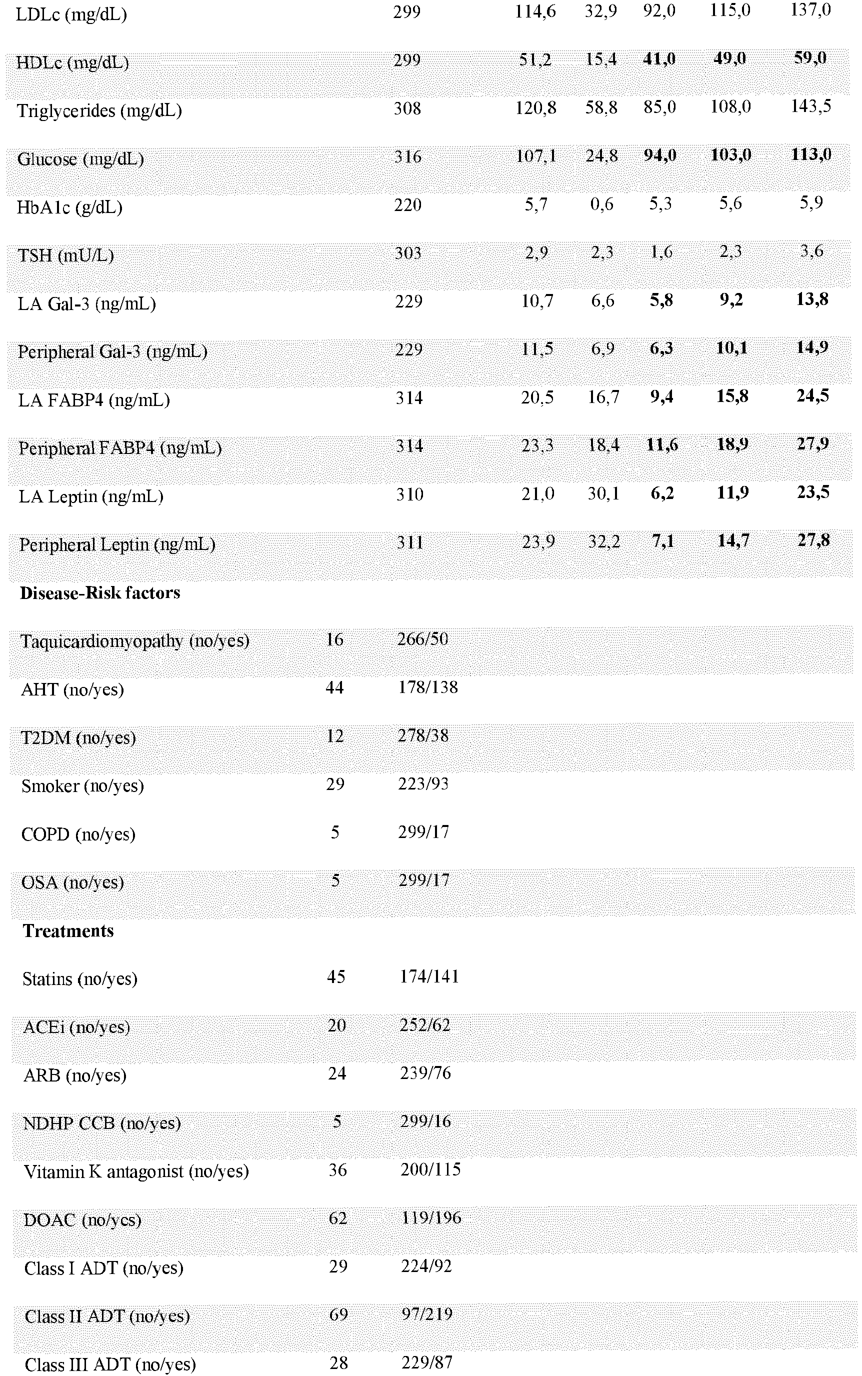

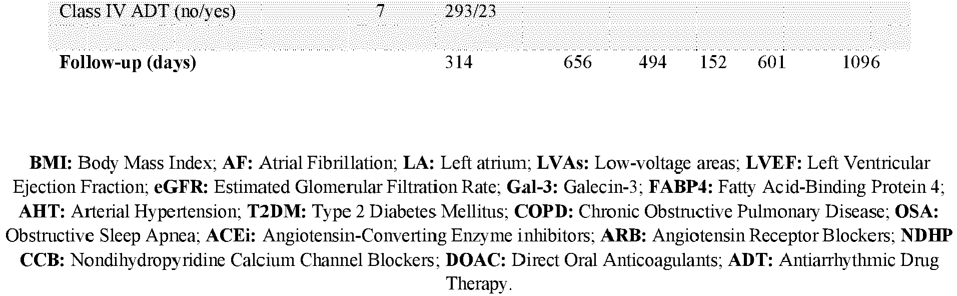

### Example 2.2. Extent of low-voltage area on EAM

Out of the 316 patients, 73 presented LVA ≥ 10%. This group was represented by higher percentage of patients with type 2 diabetes mellitus (T2DM) and arterial hypertension (AHT), larger LAA and older age. They presented a trend towards lower estimated glomerular filtrated rate (eGFR). See **Table 2** showing clinical characteristics of patients regarding LVAs < or ≥ 10%.

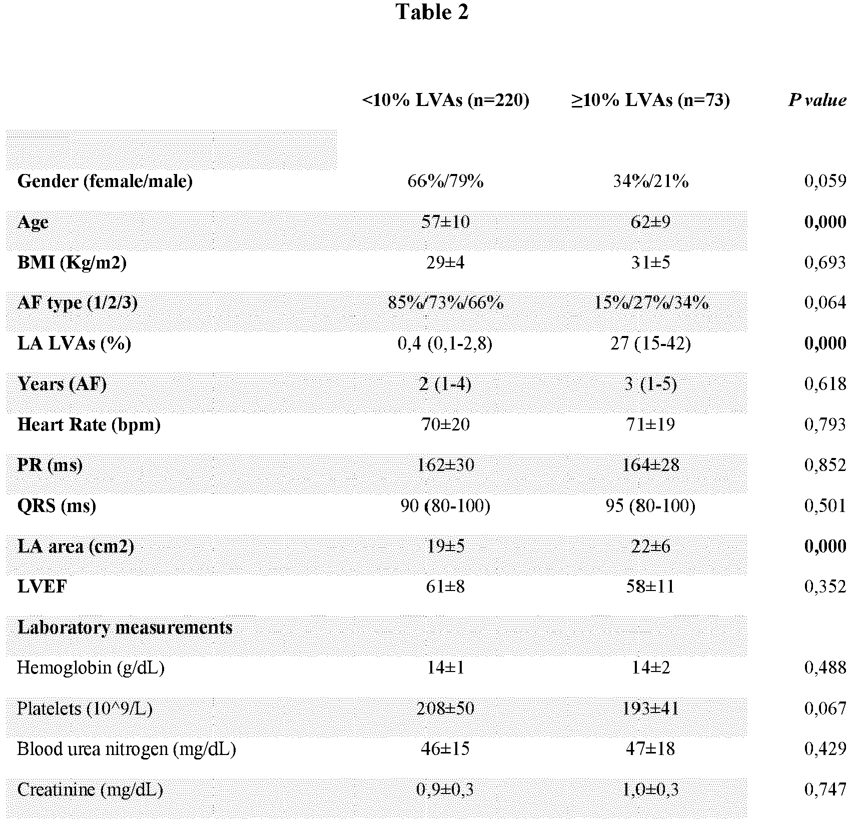

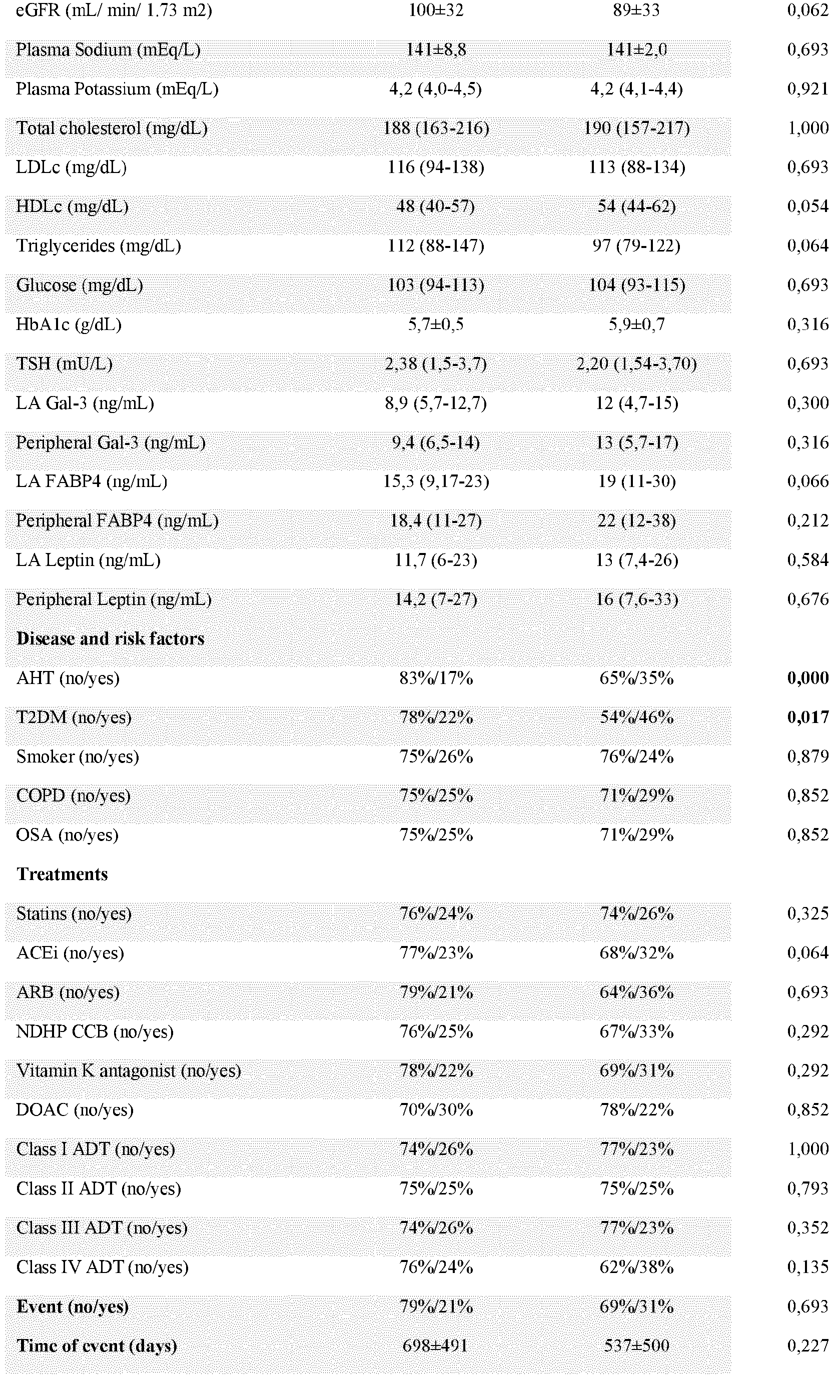

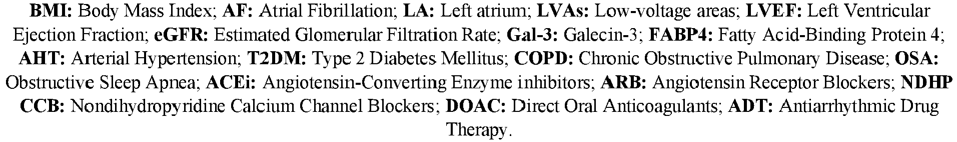

Multivariate analysis revealed gender (female), atrial or peripheral Gal-3 and FABP4 levels, LAA and AF type (long-standing persistent pattern) independent predictors for LVA ≥10%. Please refer to **Table 3a** and **3b** showing the logistic regression analysis: dependent variable LVAs < or ≥ 10%.

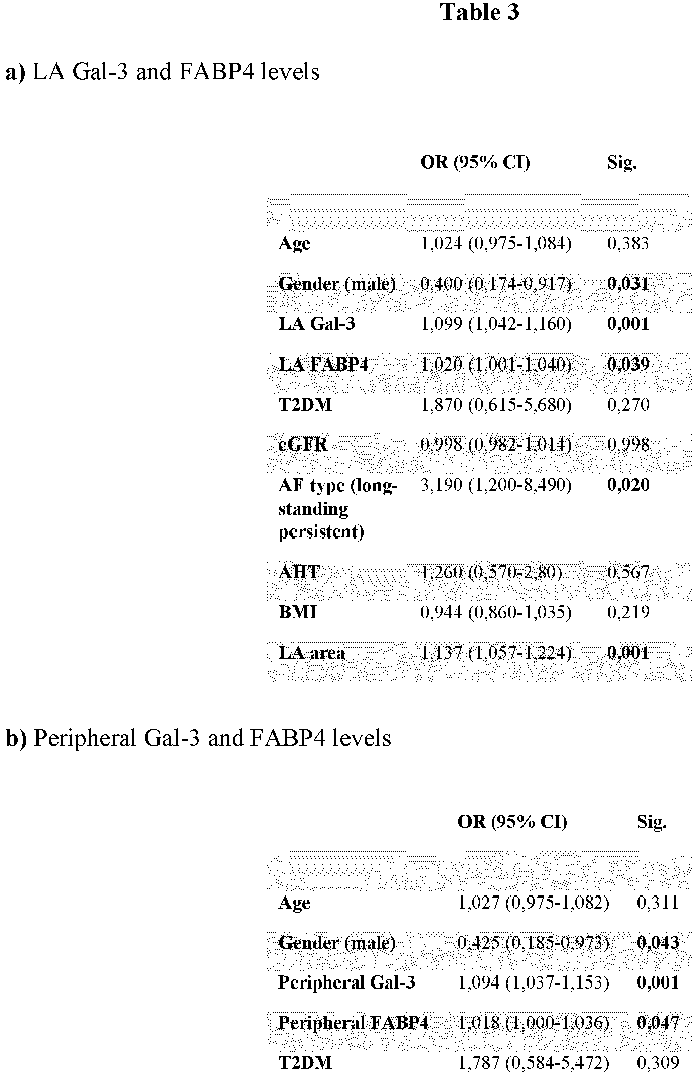

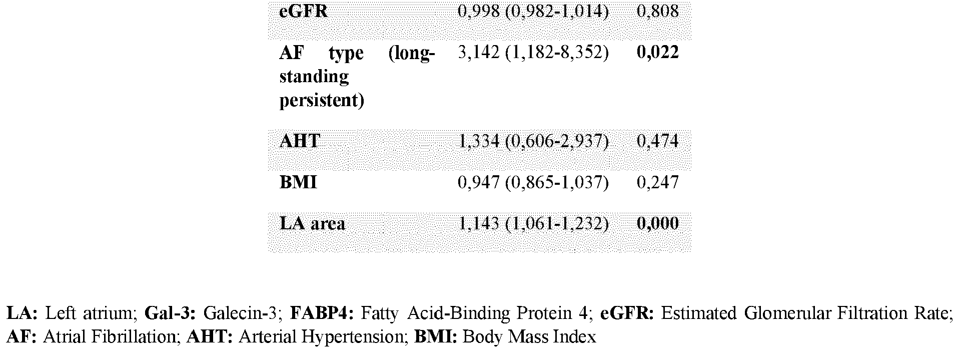

### Example 2.3. Recurrence after AF catheter ablation

Our population presented 60% freedom of recurrence during follow-up. Patients with long-standing persistent AF pattern had recurrence in a shortened period of time as compared to patients with persistent and paroxysmal pattern (496±442 vs 655±519 vs 782±467 days; p<0.001).

A score was made by assigning 1, 2, 3 points if the AF pattern was paroxysmal, persistent or long-standing persistent, respectively. Then, 1 point was added to AF type if peripheral plasma FABP4 levels were greater than 20 ng/mL and another point in case of Gal-3 greater than 10 ng/mL. This led to a final score of up to 5 points in which a punctuation of 1 was assigned to paroxysmal AF and 5 points was assigned to long-standing persistent AF with peripheral levels of FAPB4 and Gal-3 greater than 20 ng/ml and 10 ng/ml, respectively.

The Kaplan Meier showed that AF type, AF type+FABP4, AF type+Gal-3 and AF type+FABP4+Gal-3 stratified patients regarding freedom from recurrence **(****Figure 1****).** As shown in the Kaplan Meir curves, the present score reclassified patients in a more efficient manner than the conventional AF classification; the AF recurrent was detected in 27% of patients classified as score 1 and in 88% of patients classified as score 5 **(****Figure 2****),** specifically, the Cox regression analysis determined that AF type combined with FABP4 and Gal-3 were able to discriminate the population according the highest risk for AF recurrence after ablation 5.82 (1.95-17.39), p<0.001. The model with AF type alone showed a higher risk for recurrent AF in those patients with long-persistent AF 2.80 (1.75-4.49), the model with AF type and added FABP4 levels > 20 ng/mL showed a higher risk for AF recurrent in those patients with long-standing persistent AF with higher FABP4 levels than 20 ng/mL 5.25 (2.52-10.91) and the model with AF type added Gal-3 showed a higher risk for AF recurrent in those patients with long-standing persistent AF with higher Gal-3 levels than 10 ng/mL 2.81(1.33-5.94). Same procedure was performed with LVA ≥10% and FABP4 or Gal-3 and AF type. An additional point was added in patients with LVA ≥10% (Supplementary **Figure 1****).** The Log-rank showed statistical significance on every stratification groups. We have included in a Cox regression multivariate analysis the following variables (age, AHT, gender, LA area, T2DM, eGFR, AF type+Gal-3+FABP4). It showed that the combined indicator (AF type+Gal-3+FABP4) had the highest odds ratio (6,200 (1,847-20,817)) with statistical significance **(Table 4)** for AF recurrence.

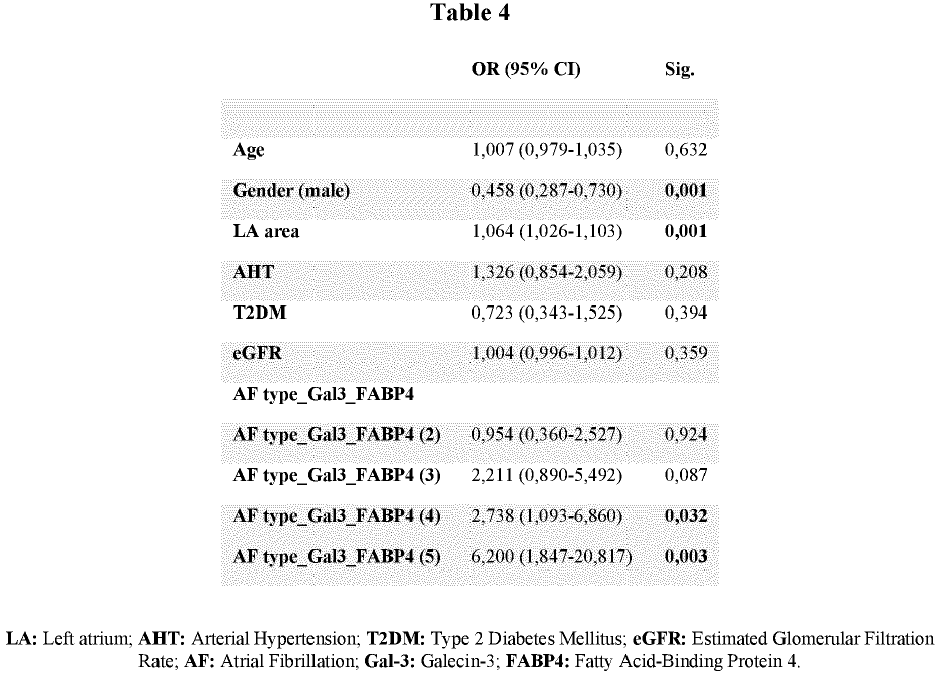

### Example 2.4. FABP4 effects on human atrial fibroblasts

The "in vitro" assay showed a reduction of fibroblasts proliferation rate after treatment with FABP4 at 10 ng/mL (46.69 ± 13.63 arbitrary units (a.u.) control vs 39.43 ± 12.88 a.u. FABP4 10 ng/mL, p=0.0164). However, the highest FABP4 concentration (100 ng/mL) enhanced the proliferation rate of human atrial fibroblasts (46.69 ± 13.63 a.u. control vs 54.91 ± 13.31 a.u. FABP4 at 100 ng/mL, p=0.0266) after 8 hours of treatment **(****Figure 3****).**

## Claims

1. *In vitro* method for predicting the response of patients suffering from atrial fibrillation (AF) to catheter ablation treatment which comprises: a) Measuring the concentration level of at least [Gal-3 and FABP4] in a biological sample obtained from the patient, and b) wherein if the concentration level of at least [Gal-3 and FABP4] is statistically higher as compared with a pre-established threshold concentration level, it is an indication that the patient will not respond to catheter ablation treatment.

2. *In vitro* method for the prognosis of patients suffering from AF which comprises: a) Measuring the concentration level of at least [Gal-3 and FABP4] in a biological sample obtained from the patient, and b) wherein if the concentration level of [Gal-3 and FABP4] is statistically higher as compared with a pre-established threshold concentration level, it is an indication that the patient has a bad prognosis, wherein bad prognosis indicates the presence of underlying fibrotic scar and arrhythmia recurrence after catheter ablation treatment.

3. *In vitro* method, according to any of the previous claims, wherein the method is carried out in persistent AF patients in which a catheter ablation is indicated for treatment.

4. *In vitro* method, according to any of the previous claims, which comprises a) Measuring the concentration level of at least [Gal-3 and FABP4] in a biological sample obtained from the subject, b) processing the concentration level values in order to obtain a risk score and c) wherein if the concentration level is statistically higher as compared with a pre-established threshold concentration level, it is an indication that the patient will not respond to catheter ablation treatment or has a bad prognosis.

5. *In vitro* method, according to any of the previous claims, wherein the patient is suffering from AF.

6. *In vitro* method, according to any of the previous claims, wherein the biological sample is a minimally-invasive biological sample.

7. *In vitro* method, according to any of the previous claims, wherein the biological sample is plasma, blood or serum.

8. *In vitro* use of at least [Gal-3 and FABP4] for predicting the response of patients suffering from AF to catheter ablation treatment, or for the prognosis of patients suffering from AF.

9. *In vitro* use, according to claim 8, in persistent AF patients in which a catheter ablation is indicated for treatment.

10. *In vitro* use, according to any of the claims 8 or 9, in a minimally-invasive biological sample obtained from the patient.

11. *In vitro* use, according to any of the claims 8 to 10, in plasma, blood or serum obtained from the patient.

12. Kit comprising:
a. Reagents or tools for obtaining a minimally-invasive sample from the patients, and
b. Reagents or tools for measuring the concentration level of at least [Gal-3 and FABP4].

13. Kit, according to claim 12, comprising:
a. Reagents or tools for obtaining plasma, blood or serum from the patients, and
b. Reagents or tools for measuring the concentration level of at least [Gal-3 and FABP4].

14. Use of the kit, according to any of the claims 12 or 13, for predicting the response of patients suffering from AF to catheter ablation treatment, or for the prognosis of patients suffering from AF.
